# EUROPEAN PATENT APPLICATION

(11) **EP 4 748 297 A1**
(43) Date of publication of application: **27.05.2026**
(21) Application number: 24020335.6
(22) Date of filing: 24.11.2024
(51) Int. Cl.: A61B 3/12

(54) **EMBEDDED RETINAL IMAGING COMPACT CAMERA MODULE WITH THIRD PARTY APPLICATION DEVELOPMENT API**

(71) Applicant: Burhan, Deniz, 34794 Cekmekoy Istanbul (TR); Lynda Sezi, Deniz, Çekmeköy Istanbul (TR); Toslak, Devrim, 07070 Antalya (TR)
(72) Inventor: Burhan, Deniz, 34794 Cekmekoy Istanbul (TR); Lynda Sezi, Deniz, Çekmeköy Istanbul (TR); Toslak, Devrim, 07070 Antalya (TR)

(57) **Abstract**

This invention relates to a compact camera module (CCM) for capturing retinal images, built into or embedded in various electronic devices such as smartphones, tablets, laptops, and wearables. The system is designed to facilitate AI-driven systemic disease detection by providing high-quality retinal images to third-party developers through an API. Alternatively, the system may include a built-in AI disease diagnosis application within the CCM software, eliminating the need for third-party integration. The retinal images can be analyzed by AI to detect early signs of systemic diseases, such as diabetes, hypertension, and cardiovascular conditions, thus making advanced health diagnostics accessible, especially in underdeveloped areas. The system features a dual-purpose objective lens for both optical and illumination paths. It is optimized for integration into portable consumer electronics with space constrains, enabling widespread usage and access to diagnostics.

## Description

### Background of the Invention

This invention relates to compact retinal imaging systems embedded into portable consumer electronics with space constraints. Traditional retinal imaging devices are typically bulky, expensive, and require professional operation in medical centers, limiting their accessibility, particulary in underdeveloped areas. Furthermore, these devices are often not integrated with AI-based systemic disease detection algorithms, which could analyze retinal images to provide diagnistic insights for conditions such as diabetes, hypertension, and cardiovascular diseases.

The present invention addresses these limitations by offering a compact, easy-to-integrate solution that captures high-quality retinal images. These images can be accessed via an API, enabling third-party developers to integrate their own AI models for systemic disease detection. Alternatively, the system may include a built-in AI diagnostic application, allowing image analysis directly through the software controlling the CCM, without requiring third-party applications.

Designed for integration into portable consumer electronics with space constraints such as smartphones and wearables, the CCM enables non-invasive, real-time systemic disease detection. This low-cost, accessible tool can be used both in healthcare environments and at home, eliminating the need for bulky equipment or visits to medical centers or hospitals.

In addition to flexible software configurations, the system may support multispectral illumination for enhanced imaging, or alternatively, white light illumination can be used. The novelty of this invention lies in the compact design that integrates a dual-purpose objective lens, multispectral imaging, folded optical path and API for AI functionality or AI functionality into a single fundus camera system. This combination enables the early detection of systemic diseases through retinal imaging and AI-driven analysis, offering a portable and accessible diagnostic tool integrated into portable consumer electronics.

The combination of a compact design, versatile illumination options, and the ability for developers or integrated AI to perform image analysis provides a novel, non-obvious solution, distinct from conventional systems that rely on proprietary, closed setups

### Summary of the Invention

The present invention is a compact camera module (CCM) designed for integration into portable portable consumer electronics with space constraints, such as smartphones and wearables, to capture retinal images for AI-driven systemic disease detection. The CCM may provide an API that allows third-party developers to take, access, and retrieve these retinal images and develop their own AI models for diagnostic purposes, enabling external innovation. In another alternative configuration, the system may provide a proprietary application for disease diagnostics, functioning without the need for third-party developer access. The module itself does not perform processing or analysis; it simply captures data, which can then be utilized for either third-party development or can be handled internally within the device depending upon the version of the CCM.

The optical system includes one objective lens and a lens group after the aperture, with optional advanced technologies like aspheric lenses or adaptive optics for enhanced image quality. A multispectral illumination system provides light across 2 to 8 wavelengths, ensuring comprehensive image capture for AI-driven analysis. In another version, instead of multispectral illumination, the system could also use a color sensor with white light illumination instead of a monochromatic sensor with multispectral illumination.

The novelty of the present invention arises from the combination of a dual-purpose objective lens, multispectral imaging, folded optical design, and API for AI functionality or AI functionality within the system. This combination, miniaturized into a compact camera module (CCM) that fits within tiny devices, allows for early diagnosis of systemic diseases manifested by retinal changes. By integrating these advanced features into a compact form factor, the invention offers a powerful diagnostic tool that brings medical-grade imaging capabilities into tiny consumer electronics such as smartphones, tablets, laptop computers and wearables.

The system's software utilizes the host device's existing operating system, power, and connectivity, allowing for integration without the need for standalone systems. This provides a low-cost, portable, and accessible diagnostic tool that can be used in both clinical and home settings.

By combining compact design, API flexibility, and the ability for developers to create custom AI tools, this invention offers a novel and non-obvious solution for systemic disease detection, distinguishing it from conventional closed-system methods.

### Embodiments

- Integration with Devices: The compact retinal imaging system is designed to be integrated into a variety of portable consumer devices, ensuring accessibility to advanced retinal imaging. The compact camera module (CCM) can be embedded in devices such as smartphones, tablets, laptops, smart wearables, and even televisions, allowing for widespread use in different environments. This integration enables users to capture retinal images through familiar devices, promoting early diagnosis of systemic diseases and expanding the range of applications for both diagnostic and non-diagnostic purposes like identification.
- Optical Path Configurations: The optical path can be configured in either a folded or non-folded manner. The folded configuration employs a light-folding element, such as a prism or mirror, to redirect the optical axis, enabling a compact layout suitable for slim devices. The non-folded configuration utilizes a direct optical path arrangement, providing a layout for devices with more internal space. The optical system includes one objective lens positioned at the front of the system, with a lens group positioned after the aperture, meaning the optical system consists of more than one lens element. While the system may incorporate advanced technologies such as aspheric lenses, metalenses, microlens arrays, and adaptive optics to achieve high image quality and minimal aberrations, these are each considered optional embodiments.
- **Imaging System:** The multispectral imaging system is capable of capturing images across 2 to 8 different wavelengths to provide detailed visualization of retinal structures. The system utilizes illumination sources such as fiber optic cables or micro LEDs, each configured to emit specific wavelengths for enhanced diagnostic capability. In one configuration, the system captures red, green, blue, and near-infrared (NIR) wavelengths, enabling comprehensive diagnostic assessment of retinal health and systemic conditions. The optical and illumination elements of the system are specifically designed and optimized to provide suitable raw or processed data for AI-driven analysis, particularly in diagnosing ocular and systemic diseases. It offers insights into different retinal layers, facilitating the detection of conditions that may not be visible with standard imaging techniques.

**In an alternative embodiment,** the system may incorporate a **multispectral sensor** that captures multiple wavelengths simultaneously without the need for sequential illumination. This embodiment reduces the possibility of motion artifacts and enables real-time capture of both visible and NIR wavelengths. The multispectral sensor can be optimized for specific bands, such as RGB and NIR, allowing for enhanced flexibility in diagnostic approaches. The simultaneous capture of these wavelengths provides a streamlined solution for real-time applications, ensuring precise alignment across spectral bands while maintaining sensitivity and diagnostic accuracy.

In another embodiment, the system may utilize a color sensor with white light illumination instead of multispectral imaging, offering flexibility for different diagnostic approaches
- Advanced Optical Technologies: The system employs advanced optical technologies to enhance imaging capabilities, including metalenses, which reduce lens thickness while maintaining high resolution, and adaptive optics, which allow real-time adjustments to correct optical aberrations and improve image clarity. Lightfield technology captures depth information, enabling computational refocusing and multi-perspective analysis of retinal structures. These technologies are integrated into the CCM to provide superior image quality while maintaining a compact form suitable for integration into mobile devices.
- Autofocus and Image Stabilization Mechanisms: The system incorporates autofocus and image stabilization mechanisms to ensure precise imaging of the retina. A MEMS actuator dynamically controls the position of the lens elements, enabling fine adjustments to maintain focus and stabilize the image. Alternative mechanisms, such as voice coil motors, liquid lenses, and piezoelectric actuators, may be used for high-precision adjustments. These systems are critical for maintaining image quality, particularly in portable devices where motion and vibration may be factors. **In another embodiment,** the system may include manual pupil tracking software, allowing users to manually track and align the pupil for more precise image capture.
- Illumination System: The illumination system provides uniform and controlled lighting of the retina through various methods, including fiber optic cables, micro LEDs, or holographic light sources. The system can deliver light at specific wavelengths to obtain optimized images for diagnostic purposes, particularly for AI models. Each illumination source is precisely aligned with the optical path to avoid interference and ensure high-quality imaging. The design allows for multispectral illumination without the need for additional lens components, reducing the overall size and complexity of the system. In an alternative embodiment, the system may utilize white light illumination instead of multispectral illumination, offering a simplified yet effective approach for certain diagnostic applications.
- Data Handling: The system may provide both raw and processed retinal data, offering secure API access for third-party developers to build custom applications for healthcare, research, and other fields. In an alternative embodiment, the system may operate without third-party API access, instead utilizing a proprietary application within the device for processing and analysis of the retinal data, enabling internal diagnostic capabilities without the need for external development.
- Multispectral Image Layering Embodiment: In this embodiment, the system is configured to capture images using its multispectral imaging capabilities, where light in the range of 2 to 8 wavelengths is employed to illuminate the retina. Each captured image corresponds to a specific wavelength or spectral band. The images may be layered on top of each other in a manner that preserves the unique information from each spectral band. This layered approach enables a more detailed analysis by maintaining the distinct characteristics of each wavelength, providing a comprehensive view of the retinal structure and facilitating more accurate diagnostics or data interpretation by third-party applications. If the multispectral imaging embodiment is present, these processes are applied to achieve enhanced diagnostic accuracy.
- Autoalignment and Autocapture Embodiment: The system may comprise autoalignment and autocapture functionalities to streamline the imaging process. The autoalignment mechanism, if used, is operable to automatically adjust the position of the system to ensure proper alignment of the retina within the field of view for optimal image capture. In embodiments that include autocapture, the system may automatically initiate image capture at an optimal moment, determined by parameters such as focus, illumination, and retinal positioning. These features, when employed, minimize the need for manual adjustment or intervention, enhancing usability and ensuring consistent image quality, particularly in portable or handheld devices.

### Brief Description of Drawings

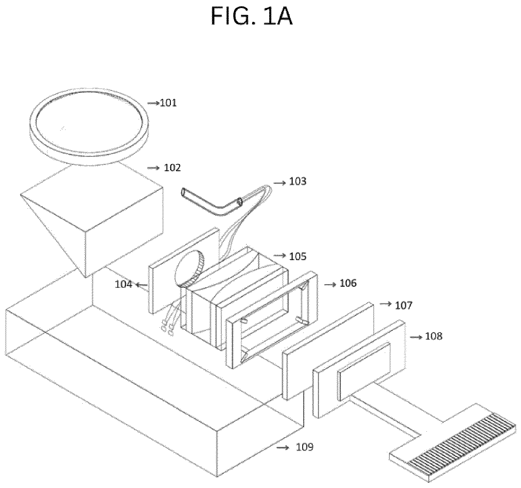

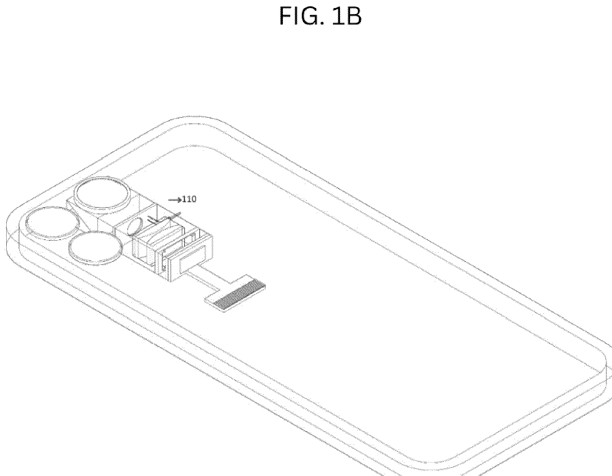

**FIG. 1A** **- Compact Folded Lens System with Prism or Mirror:** FIG. 1A is an exploded illustration of a compact camera module featuring a folded lens system that integrates multiple components for retinal imaging and diagnostic applications. The system comprises:
   - **101 - Objective Lens:** This lens first collects light reflected from the retinal surface and directs it through the optical system, minimizing aberrations and providing high-resolution imaging across the entire field of view. Additionally, it directs illumination onto the retina by focusing light from the system's light sources through its optical elements.
   - **102 - Prism or Mirror:** The prism serves as a light-folding element that redirects the optical path, allowing the module to maintain a compact form factor suitable for integration into slim mobile devices. It deflects incoming light from the objective lens towards the subsequent optical components.
   - **103 - Multispectral Illumination System:** In the multispectral illumination embodiment, the system includes light sources emitting four wavelengths: red, green, blue (RGB), and near-infrared (NIR). The multispectral illumination is delivered through precisely aligned fiber optics, ensuring uniform lighting conditions essential for capturing detailed retinal images.
   - **104 - Aperture:** Positioned after the prism, the aperture controls the amount of light entering the optical system. It regulates light intensity and depth of field, contributing to the overall clarity and quality of the captured images.
   - **105 - Lens System:** This consists of multiple lens elements designed to focus light onto the image sensor. The configuration ensures sharp, high-quality retinal images, suitable for AI-driven diagnostic processes.
   - **106 - MEMS Actuator:** The MEMS actuator controls the position of the camera lens system, enabling fine adjustments for focus and stabilization. It dynamically responds to changes according to the refractive status of the eye, ensuring that the system maintains optimal focus on the retinal surface.
   - **107 - Image Sensor:** The image sensor captures the focused light and converts it into digital image data for further processing. The sensor may be either monochromatic, multispectral or color, depending on the specific configuration of the system.
   - **108 - Integration Module:** This component facilitates the connection of the camera module onto the motherboard of the host device, ensuring stable connectivity and functionality within the consumer device.
   - **109 - Module Housing:** The housing protects and stabilizes all optical and electronic components, providing a robust environment that maintains the module's alignment and functionality.
**FIG. 1B** **- Module Integration with Device - Top View:** FIG. 1B illustrates the integration of the compact camera module into the host device, shown in a top-down view:
   - **110 - Integrated Camera Module:** The camera module is embedded into the device. This placement within the device chassis ensures optimal functionality while maintaining the device's overall form factor and design.
**FIG. 2A** **- Non-Folded Lens System Without Prism:** FIG. 2A shows a camera module similar to FIG. 1A but without a prism. This configuration represents a non-folded optical path, which can be utilized in devices with more internal space. The components include:
   - **201 - Objective Lens:** Similar to FIG. 1A, this lens collects and directs light through the optical system, ensuring minimal aberrations and high-quality imaging. Additionally, it directs illumination onto the retina by focusing light from the system's light sources through its optical elements.
   - **203** - **Multispectral Illumination System:** The same illumination system as in FIG. 1A, emitting RGB and NIR wavelengths for detailed visualization of retinal structures.
   - **204 - Aperture:** Controls light entry into the optical system, managing depth of field and preventing overexposure.
   - **205 - Lens System:** Composed of multiple lens elements for focusing light onto the image sensor, ensuring high-resolution image capture.
   - **206 - MEMS Actuator:** Provides fine adjustments to maintain focus, compensating for any movement or misalignment. It dynamically may respond to changes according to the refractive status of the eye, ensuring that the system maintains optimal focus on the retinal surface.
   - **207 - Image Sensor:** Captures the focused light and converts it into digital data for image processing and analysis. It may be either monochromatic or color sensor.
   - **208 - Integration Module:** Connects the camera module to the motherboard of the host device, facilitating stable data transmission and power supply.
   - **209 - Module Housing:** Encloses the entire system, protecting it from external factors and ensuring stable performance within the host device.

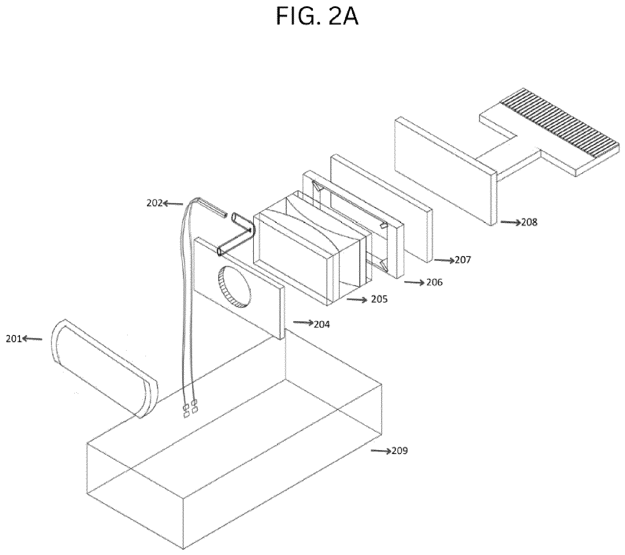

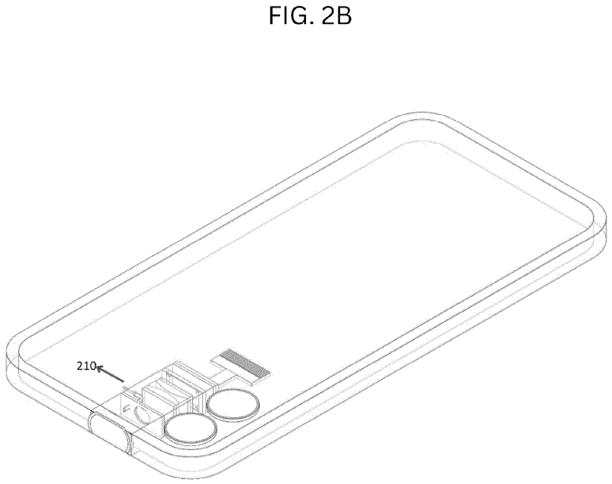

**FIG. 2B** **- Module Integration with Device - Side View:** FIG. 2B provides a side view of the integrated camera module within the host device:
   - **210 - Integrated Camera Module:** Similar to FIG. 1B, this illustration shows the placement of the camera module within the device, indicating its alignment and connection to the device's internal components, ensuring that it functions seamlessly as part of the overall device system.

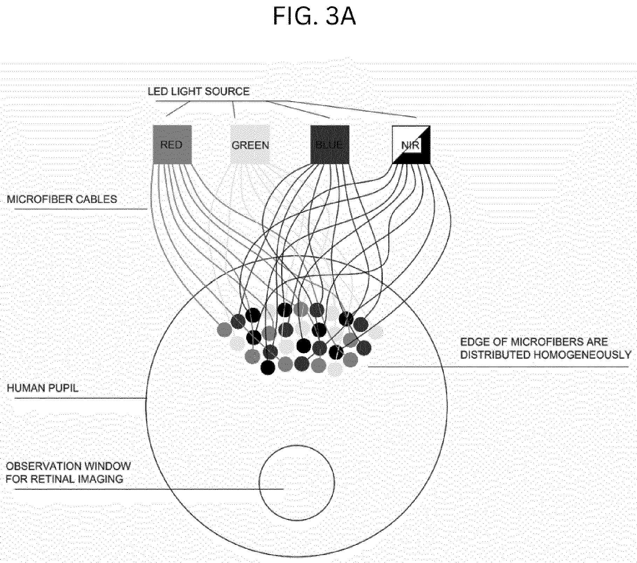

**FIG. 3A** **- Multispectral Illumination System with Fiber Optics:** FIG. 3A is a detailed illustration of the multispectral illumination embodiment, designed for a compact camera module (CCM) used in retinal imaging.
   - **301 - LED Light Source:** The LED light sources emit light in four spectral bands: Red, Green, Blue (RGB), and Near-Infrared (NIR). Each LED corresponds to a specific wavelength and is responsible for illuminating the retina through dedicated microfiber cables. The multispectral illumination provides comprehensive data by capturing retinal images across various wavelengths, which is critical for diagnostic applications.
   - **302** - **Microfiber Cables:** These microfiber cables transmit light from the LED light sources to the retina. Each color channel (Red, Green, Blue, NIR) is connected to a separate set of fibers, ensuring that each wavelength illuminates the retina evenly. The cables are designed to preserve the intensity and quality of the light as it is transmitted, ensuring uniform illumination across the retina.
   - **303 - Human Pupil:** The human pupil is the opening through which light from the LED sources enters the eye and reaches the retina. The light, transmitted through the microfiber cables, passes through the pupil and illuminates the retinal surface. The system ensures that the pupil is adequately illuminated without causing glare or reflections that could degrade image quality.
   - **304 - Observation Window for Retinal Imaging:** This observation window captures the reflected light from the illuminated retina. Once the retina is illuminated by the multispectral system, the light reflected off the retinal surface is directed back through the observation window to the image sensor.
   - **305 - Edge of Microfibers Distributed Homogeneously:** The edges of the microfiber cables are distributed homogeneously around the illumination window, ensuring uniform light distribution across the retina. This homogeneous distribution is essential for preventing any uneven illumination that could result in imaging artifacts. It also ensures that all regions of the retina are equally illuminated, which improves the diagnostic accuracy of the captured images.

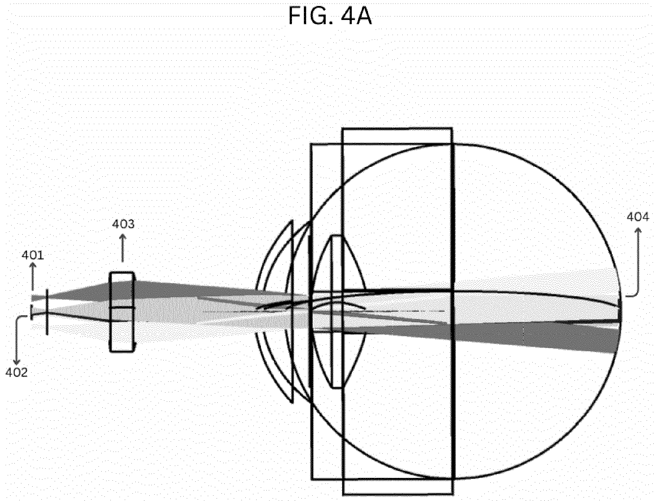

**FIG. 4A** **- Overlapping of Multispectral Illumination and Optical Path on Retina:** FIG. 4A is a 3D Layout showing a multispectral illumination and optical path system for retinal imaging. RGB and NIR wavelengths illuminate the retina, while the optical path captures reflected light. The objective lens serves both illumination and imaging functions, ensuring successful retinal imaging by overlapping the illumination and optical paths
   - **401 - Multispectral Illumination Path:** The layout demonstrates how RGB and NIR wavelengths, represented by the ends of fiber cables, follow the illumination path. These wavelengths fall on the retina, effectively illuminating it for imaging.
   - **402 - Optical Path:** The light reflected from the illuminated retina is captured along the optical path and directed back through the system. This ensures that the information is successfully collected by the optical elements.
   - **403 - Objective Lens:** The objective lens serves a dual purpose in this system. It directs the illumination from its back surface toward the retina and also gathers the light reflected from the retina, capturing the necessary data for imaging.
   - **404 - Retina:** Both the optical path and the multispectral illumination path overlap on the retina, ensuring that the retina is successfully illuminated and imaged, which is critical for accurate multispectral retinal imaging.

### Detailed Description of the Invention

### A. Optical System:

The optical system of the compact camera module (CCM) includes both folded and non-folded configurations. The folded configuration may use a prism or mirror to maintain compactness for space-constrained devices like smartphones and wearables, while the non-folded configuration is used when the optical path can remain direct.

The dual-purpose objective lens in the CCM performs both optical focusing for image capture and illumination within the compact space of the module. The back surface of the objective lens focuses illumination light onto the pupil, eliminating the need for additional focusing optics. Optional advanced optical elements, such as aspheric lenses and metalenses, may be included to enhance image quality and reduce aberrations but are not required for the system's core functionality.

### B. Illumination System:

In the multispectral illumination embodiment, the system uses multispectral light delivered via fiber optic cables, micro LEDs, or laser-based sources, supporting 2 to 8 wavelengths (e.g., red, green, blue, near-infrared). This enhances image quality for use in third-party AI applications by providing more detailed retinal imaging. The multispectral light is uniformly distributed using microfiber cables around the illumination window, ensuring consistent illumination across the retina. This avoids overexposure or underexposure and improves image reliability. The light is focused onto the pupil by the back surface of the objective lens, eliminating the need for additional optics. In an alternative embodiment, a color sensor may be used with white light illumination instead of multispectral light, simplifying the system while still enabling effective retinal imaging.

### C. Imaging System and Autocapture:

The imaging system is designed to capture retinal images using either a monochromatic or color sensor, depending on the specific configuration of the system. In the multispectral imaging embodiment, the system captures images across multiple wavelengths to visualize retinal structures. Autocapture, if used, along with autofocus, may be managed by the software within the host device's OS. Algorithms, such as contrast detection, assess image clarity and focus, and in embodiments utilizing autocapture, the system may trigger image capture at the optimal moment without manual intervention.

### D. Data Processing:

The system provides images in either raw or processed formats. Processed data may include noise reduction, contrast adjustment, and false coloring. In one embodiment, developers can access these images via the API to develop custom applications for systemic disease detection. In another embodiment, an AI diagnostic application may be developed to analyze the data captured by the CCM software and perform diagnoses without the need for third-party integration.

### E. Integration with Various Devices:

The CCM is designed for easy integration into portable consumer devices with space contraints such as smartphones, tablets, laptops, and wearables. Its modular design allows it to be embedded directly onto the motherboard or used as a detachable module, offering flexibility across different fields.

An API may be provided to allow developers to capture, access and retrieve images, while the CCM's software integrates with the host device's OS to optimize performance and communication with the hardware.

### F. API and AI Integration:

The CCM offers two versions for AI-driven systemic disease detection. In one embodiment, the CCM provides an API that allows third-party developers to capture, access and retrieve retinal images, either in raw or processed form, to develop their own AI-based diagnostic applications. The CCM itself does not process or analyze the images; it serves as a data collection tool for external AI models.

In another embodiment, the CCM includes an integrated AI diagnostic application, which processes the retinal images directly within the device for systemic disease detection. In this configuration, the CCM performs the analysis internally without the need for third-party integration, offering a complete, self-contained diagnostic solution.

## Claims

1. A compact fundus camera module (CFM) integrated into portable consumer electronic devices to enable third-party developers to acquire retinal images for developing AI-driven disease detection applications, the system comprising:
• **a.** An optical system configured to fit within a thickness less than 9 mm, allowing for integration into smartphones, tablets, laptops, smart wearables, or other portable consumer electronic devices with space constraints;
• **b.** An illumination system comprising:
∘ **i.** A light source that, if multispectral, supports 2 to 8 wavelengths, or alternatively, a white spectrum light source.
∘ **ii.** An illumination window positioned to direct light onto the back surface of a dual-purpose objective lens, which handles both illumination focusing and optical focusing for image capture;
o **iii.** Wherein the back surface of the objective lens is configured to focus the illumination light onto the retina, eliminating the need for additional focusing optics in the illumination path;
• **c.** An image sensor positioned to capture light reflected from the retina after passing through the optical system;
• **d.** A software interface provided within the operating system's camera framework, enabling third-party developers to initiate image capture, access, and retrieve retinal images through the CCM for diagnostic applications, systemic disease analysis, real-time health monitoring, and other uses, without requiring standalone software on the CFM.

2. The system of Claim 1, wherein the API provided in the operating system's camera framework enables third-party developers to request either raw or processed retinal image data.
• Both processed and raw retinal images can be used for similar applications, such as real-time retinal health monitoring, early disease detection, non-medical purposes including biometric identification or other security-related applications.
• In another embodiment, raw retinal image data can be used for specialized analysis, including medical-grade diagnostics, clinical research, or advanced AI model development.

3. **a.**The system of Claim 1, wherein the illumination system, in the multispectral imaging embodiment, further comprises:
• Multiple sets of microfiber cables, each set dedicated to a specific wavelength, including at least Red, Green, Blue (RGB), and Near-Infrared (NIR);
• A plurality of LED light sources, each corresponding to one of the sets of microfiber cables;
• Wherein the illumination window holds all microfiber cables together in a uniform arrangement, ensuring even illumination across the retina for multispectral imaging.
**b.**The system of Claim 1, wherein the illumination system, in the white light imaging embodiment, further comprises:
• A single fiber cable or LED light source emitting white spectrum light;
• An illumination window housing the light source, ensuring uniform illumination across the retina for imaging.

4. The system of Claim 3, in the multispectral imaging embodiment, wherein:
• a. The LED light sources are sequentially activated in response to a user command, such as pressing an image capture button, or automatically through an autocapture feature, to illuminate the retina with each color in a predetermined sequence;
• b. The sequential activation is optimized for the compact form factor, minimizing heat generation and power consumption while maintaining high-quality multispectral imaging.

5. The optical and illumination system for the compact fundus camera module (CFM) of Claim 1 comprising:
• **a.** An objective lens configured to focus light onto the retina for imaging, wherein the back surface of the objective lens is additionally configured to focus illumination light onto the pupil, eliminating the need for additional focusing optics within the illumination path;
• **b.1.** If multispectral imaging is employed, a uniformly distributed array of microfiber cables transmits multispectral illumination light simultaneously to the retina via the back surface of the objective lens, providing uniform illumination for multispectral imaging;
**b.2.** If white light illumination is utilized, a fiber cable transmits white spectrum light to the retina via the back surface of the objective lens, providing uniform illumination;
• **c.** An optical path configured to capture the reflected light from the retina for imaging purposes, wherein the optical path is free of additional focusing elements in the illumination system;
• **d.** Wherein the optical system achieves a total track length of less than 25 mm thickness, enabling integration into portable consumer electronic devices with space constraints while maintaining image quality suitable for medical-grade retinal imaging.

6. A method for operating the compact fundus camera integrated into a portable consumer electronic device with space constraints, comprising:
• a. Activating the illumination system to direct light onto the back surface of the objective lens;
• b. Focusing the light onto the retina using the back surface of objective lens;
• c. In the multispectral imaging embodiment, processing the captured images to produce a composite multispectral retinal image, or leaving them in raw form;
• d. In the API embodiment, making the processed or raw images available to third-party applications through the API for analysis or other purposes.

7. A method for integrating a compact fundus camera into a portable consumer electronic device with space constrain, comprising:
• **a.** Designing a compact fundus camera module with a volume around 1300 cubic millimeters;
• **b.** Configuring the illumination system to use the back surface of the objective lens for focusing light onto the retina;
• **c.** Adapting the optical path to fit within the form factor of the portable consumer electronic device with space constrains while maintaining medical-grade imaging capability;
• **d.** Integrating the compact fundus camera module into the portable consumer electronic device in a manner that allows for normal operation of the device's other features.

8. The system of **Claim 1,** wherein the integration of the compact fundus camera module into a portable consumer electronic device with the help of AI driven algorithms enables:
• **a.** Daily or on-demand health monitoring by end-users;
• **b.** Early detection of retinal abnormalities or systemic diseases that manifest in retinal changes;
• **c.** Longitudinal tracking of retinal health over time;
• **d.** Secure storage and transmission of retinal images for remote analysis by healthcare professionals.

9. The optical system for the compact fundus camera module (CFM) of **Claim 1,** further comprising:
• **a.** An objective lens configured to focus light reflected from the retina, where the lens may include aspheric, glass, or hybrid lenses made of any suitable material;
• **b.** A prism or mirror configured in a folded (periscope-style) optical path to fit within the form factor of mobile consumer devices with space constrains, wherein in another embodiment, the optical path may be non-folded. The prism or mirror enables the redirection of light within the optical system, allowing for a more compact design without reducing the total track length (TTL);
• **c.** An aperture positioned along the optical path to regulate the amount of light entering the system, optimizing image clarity and depth of field;
• **d.** A lens group positioned after the aperture, consisting of one or more lens elements, such as microlens arrays, light field lenses, or metalenses, configured to focus the light onto the image sensor;
• **e.** A MEMS actuator or fixed configuration of lenses for fine adjustments to focus and alignment within the optical path, depending on the embodiment;
• **f.** An image sensor behind the camera lens group, configured to capture the focused light.

10. The optical system of **Claim 9,** wherein:
• **a.** In one embodiment, the MEMS actuator, if present, is controlled by an integrated control unit responsible for adjusting the lens group to maintain optimal focus and alignment;
• **b.** In another embodiment, the autofocus mechanism, driven by the MEMS actuator, ensures that retinal images are captured with precision regardless of slight eye movements. It may also dynamically respond to changes in the refractive status of the eye, further enhancing imaging accuracy;
• **c.** In yet another embodiment, the system operates in a fixed-focus configuration without the MEMS actuator, where the lens group remains stationary and the focus is preset, suitable for environments where no dynamic adjustments are required.

11. The optical system of **Claim 10,** wherein the optical path is designed to integrate with the illumination system described in **Claim 1,** allowing uniform illumination of the retina during image capture.

12. A method for operating the optical and illumination systems of **Claim 10,** comprising the steps of:
• **a.** Sequentially activating the LED or fiber optic light sources to provide uniform illumination;
• **b.** Adjusting the focus of the optical system, either automatically via the MEMS actuator or through a fixed focus configuration, to capture high-quality retinal images;
• **c.** Transmitting the captured retinal images to the device's processing unit, where they are accessible through the API for real-time analysis by third-party developers or processed by an integrated AI diagnosis application.

13. The invention claims a system that integrates a dual-purpose objective lens, multispectral imaging, folded optical path, and API for AI functionality or AI functionality within the system into a compact camera module (CCM) for smartphones. The novelty lies in the combination of these elements and their miniaturization into a device capable of detecting systemic diseases through retinal imaging and performing AI-based diagnostics.

## Amended claims

### Amended claims in accordance with Rule 137(2) EPC.

1. A compact fundus camera module (CFM) **embedded within the housing of consumer electronic devices** to enable third-party developers to acquire retinal images for developing AI-driven systemic disease detection applications, the system comprising:
• a. An optical system (101, 108) configured to fit within a thickness less than 9 mm (109), allowing for integration **into smartphones, tablets, laptops,** or other portable consumer electronic devices with space constraints;
• b. An illumination system (103) comprising:
i. A light source that, if multispectral, supports 2 to 8 wavelengths, or alternatively, a white spectrum light source.
∘ **ii.** An illumination window (305) positioned to direct light onto the back surface of a dual-purpose objective lens, which handles both illumination focusing and optical focusing (304) for image capture;
∘ **iii.** Wherein the back surface of the objective lens (101) is configured to focus the illumination light onto the retina, eliminating the need for additional focusing optics in the illumination path;
• **c.** An image sensor (108) positioned to capture light reflected from the retina after passing through the optical system and electrically connected to the host motherboard via a flexible printed circuit (FPC) (111)
• **d. A software interface provided within the operating system's camera framework, enabling third-party developers** to initiate image capture, access, and retrieve retinal images through the CCM for diagnostic applications, systemic disease analysis, real-time health monitoring by AI , and other uses, without requiring standalone software on the CFM.

2. The system of Claim 1, wherein the **API provided in the operating system's camera framework enables third-party developers to request either raw or processed retinal image data.**
• Both processed and raw retinal images can be used for similar applications, such as real-time retinal health monitoring by AI, early disease detection, non-medical purposes including biometric identification or other security-related applications.
• In another embodiment, raw retinal image data can be used for specialized analysis, including medical-grade diagnostics, clinical research, or advanced AI model development.

3. a. The system of Claim 1, wherein the illumination system (103) , in the multispectral imaging embodiment, further comprises:
• Multiple sets of microfiber cables (301) , each set dedicated to a specific wavelength, including at least Red, Green, Blue (RGB), and Near-Infrared (NIR);
• A plurality of LED light sources, each corresponding to one of the sets of microfiber cables (302) ;
• Wherein the illumination window holds all microfiber cables together in a uniform arrangement, ensuring even illumination across the retina for multispectral imaging.(305)
b. The system of Claim 1, wherein the illumination system (103) , in the white light imaging embodiment, further comprises:
• A single fiber cable or LED light source emitting white spectrum light;
• An illumination window housing the light source, ensuring uniform illumination across the retina for imaging.

4. The system of Claim 3, in the multispectral imaging (301) embodiment, wherein:
• a. The LED light sources are sequentially activated in response to a user command, such as pressing an image capture button, or automatically through an autocapture feature, to illuminate the retina with each color in a predetermined sequence;
• b. The sequential activation is optimized for the compact form factor, minimizing heat generation and power consumption while maintaining high-quality multispectral imaging.

5. The optical and illumination system for the compact fundus camera module (CFM) of Claim 1 comprising:
• **a.** An objective lens (101) configured to focus light onto the retina for imaging, wherein the back surface of the objective lens is additionally configured to focus illumination light onto the pupil, eliminating the need for additional focusing optics within the illumination path;
• **b.1.** If multispectral imaging is employed (301), a uniformly distributed array of microfiber cables transmits multispectral illumination light simultaneously to the retina via the back surface of the objective lens, providing uniform illumination for multispectral imaging;
**b.2.** If white light illumination is utilized, a fiber cable transmits white spectrum light to the retina via the back surface of the objective lens, providing uniform illumination;
• **c.** An optical path (101, 108) configured to capture the reflected light from the retina for imaging purposes, wherein the optical path is free of additional focusing elements in the illumination system;
• **d.** Wherein the optical system achieves a total track length of less than 25 mm thickness, enabling integration into portable consumer electronic devices with space constraints while maintaining image quality suitable for medical-grade retinal imaging for AI systemic disease detection.

6. A method for operating the compact fundus camera integrated into a portable consumer electronic device with space constraints, comprising:
• a. Activating the illumination system (103) to direct light onto the back surface of the objective lens(101);
• b. Focusing the light onto the retina using the back surface of objective lens;
• c. In the multispectral imaging embodiment(103), processing the captured images (108) to produce a composite multispectral retinal image, or leaving them in raw form;
• d. In the API embodiment, making the processed or raw images available to third-party applications through the API for AI analysis or other purposes.

7. A method for integrating a compact fundus camera into a portable consumer electronic device with space constrain (110), comprising:
• **a.** Designing a compact fundus camera module with a volume around 1300 cubic millimeters;
• **b.** Configuring the illumination system (304, 305) to use the back surface of the objective lens (101) for focusing light onto the retina;
• **c.** Adapting the optical path to fit within the form factor of the portable consumer electronic device with space constraints while maintaining medical-grade imaging optimized for AI disease detection capability;
• **d.** Embedded the compact fundus camera module into the portable consumer electronic device (108) in a manner that allows for normal operation of the device's other features.

8. The system of **Claim 1,** wherein the integration of the compact fundus camera module (111) into a portable consumer electronic device with the help of AI driven algorithms enables:
• **a.** Daily or on-demand health monitoring by end-users;
• **b.** Early detection of retinal abnormalities or systemic diseases that manifest in retinal changes;
• **c.** Longitudinal tracking of retinal health over time;

9. The optical system for the compact fundus camera module (CFM) of **Claim 1,** further comprising:
• **a.** An objective lens (101) configured to focus light reflected from the retina, where the lens may include aspheric, glass, or hybrid lenses made of any suitable material;
• **b.** A prism or mirror (102) configured in a folded (periscope-style) optical path to fit within the form factor of mobile consumer devices with space constraints, wherein in another embodiment, the optical path may be non-folded. The prism or mirror enables the redirection of light within the optical system, allowing for a more compact design without reducing the total track length (TTL);
• **c.** An aperture (104) positioned along the optical path to regulate the amount of light entering the system, optimizing image clarity and depth of field;
• **d.** A lens group (105) positioned after the aperture, consisting of one or more lens elements, such as microlens arrays, light field lenses, or metalenses, configured to focus the light onto the image sensor (108);
• **e.** A MEMS actuator (106) or fixed configuration of lenses for fine adjustments to focus and alignment within the optical path, depending on the embodiment;
• **f.** An image sensor (108) behind the camera lens group, configured to capture the focused light.

10. The optical system of **Claim 9,** wherein:
• **a.** In one embodiment, the MEMS actuator (106), if present, is controlled by an integrated control unit responsible for adjusting the lens group to maintain optimal focus and alignment;
• **b.** In another embodiment, the autofocus mechanism, driven by the MEMS actuator, ensures that retinal images are captured with precision regardless of slight eye movements. It may also dynamically respond to changes in the refractive status of the eye, further enhancing imaging accuracy;
• **c.** In yet another embodiment, the system operates in a fixed-focus configuration without the MEMS actuator, where the lens group remains stationary and the focus is preset, suitable for environments where no dynamic adjustments are required.

11. The optical system of **Claim 10,** wherein the optical path is designed to integrate with the illumination system (103) described in **Claim 1,** allowing uniform illumination of the retina during image capture.

12. A method for operating the optical (101, 105) and illumination systems (103) of **Claim 10,** comprising the steps of:
• a. Sequentially activating the LED or fiber optic light sources (301) to provide uniform illumination;
• b. Adjusting the focus of the optical system, either automatically via the MEMS actuator (106) or through a fixed focus configuration, to capture high-quality retinal images;
• **c.** Transmitting the captured retinal images to the device's processing unit (111), where they are accessible through the API for real-time analysis by third-party developers or processed by an embedded AI diagnosis application.

13. The invention claims **a system that integrates a dual-purpose objective lens, multispectral imaging, folded optical path, and API for AI functionality for systemic disease detection or AI functionality within the system into a compact camera module (CCM) for smartphones, tablets or laptops.** The novelty lies in **the combination of these elements and their miniaturization into a device capable of detecting systemic diseases through retinal imaging and performing AI-based diagnostics.**
